# EUROPEAN PATENT APPLICATION

(11) **EP 4 799 575 A2**
(43) Date of publication of application: **02.09.2026**
(21) Application number: 25223053.7
(22) Date of filing: 12.12.2025
(51) Int. Cl.: A61B 7/04

(54) **ELECTRONIC DEVICE**

(30) Priority: 26.02.2025 JP 2025028786
(71) Applicant: Onkyo Corporation, Osaka-shi Osaka 542-0081 (JP)
(72) Inventor: TAKESHIMA, Yoshitada, Osaka-shi, 542-0081 (JP); YOSHIDA, Makoto, Osaka-shi, 542-0081 (JP); WADA, Hiroshi, Yawata-shi, 614-8056 (JP); MORITA, Mikio, Osaka-shi, 537-0003 (JP)
(74) Representative: HKW Intellectual Property PartG mbB

(57) **Abstract**

A stethoscope 1 includes a substrate 10, a ground plate 5 which has an arm 5b which extends substantially parallel to a direction to which the substrate 10 extends and a screw 11 which connects the substrate 10 and the arm 5b of the ground plate 5. The ground plate 5 has a base 5a which extends to a direction which is orthogonal to a direction to which the arm 5b extends. The ground plate 5 is provided at the enclosure 2 and the base 5a exposes externally. Thus, a means is provided which can prevent mixing of a hum noise by simple structure.

## Description

### TECHNICAL FIELD

The present invention relates to an electronic device.

### BACKGROUND ART

Among stethoscopes, there is a stethoscope which is called an electronic stethoscope which electronically collects a sound such as a heart sound by a sensor such as a microphone, amplifies the collected sound and makes a doctor listen to the amplified sound (for example, see patent literature 1.). Further, there is an electrocardiograph for measuring an electrocardiogram. The electronic stethoscope is approached to a human body for obtaining a biological sound (an auscultatory sound) and the electrocardiograph is approached to the human body for obtaining the electrocardiogram. Since an electronic circuitry is included in these electronic devices (the electronic stethoscope and the electrocardiograph), there is a case where noise from a surrounding environment such as a hum noise of an electric lamp line is a problem when the electronic circuitry is near the human body. These causes are the following.
(1) The electronic circuitry and its surrounding electric potential fluctuation electromagnetically combine.
(2) An input circuit of a sensor is a high impedance input circuit when the sensor is included.

In the electronic stethoscope which measures and obtains the biological sound with contact to the human body, for example, a piezoelectric element is used. Especially, in a circuit including the high impedance piezoelectric element, noise becomes conspicuous.

Like the electrocardiogram, in equipment which measures an electric potential itself of the human body, grounding is performed by an individual clip or a ground plate is provided (Paragraph 0018 of patent literature 2 describes that a ground electric pole is provided.). Herein, in the electronic stethoscope which does not measure the electric potential of the human body directly, the ground plate is not provided. However, mixing of the hum noise by approaching a high impedance amplifier to the human body cannot be avoided.

To prevent mixing of the hum noise, an applicant filed JP 2022-129826 (see patent literature 3.). Fig. 18 is a perspective diagram illustrating a stethoscope according to JP 2022-129826 (hereinafter referred to as an "conventional invention".). The stethoscope 201 includes an enclosure 202 and a ground plate 203 which is provided at the enclosure 202 and exposes externally. In the conventional stethoscope 201, the ground plate 203 is provided at the enclosure 202 and exposes externally. When a user of the stethoscope 201 is about to approach the stethoscope 201 to the human body, the user grasps the enclosure 202 by his or her hand and approaches the stethoscope 201 to the human body. Therefore, his or her hand contacts the ground plate 203 necessarily. This prevents the mixing of hum noise.

In the conventional invention, wiring against the ground plate is a structure that a wiring is soldered to an inside of a sheet metal member and the wiring is connected to an internal substrate. With this structure, in a case where the wiring is disconnected or bitten when the stethoscope is assembled, assemblability may be spoiled.

### PRIOR ART DOCUMENT

### PATENT LITERATURE

PATENT LITERATURE 1: JP 2004-242849 A
PATENT LITERATURE 2: JP 2021-078596 A
PATENT LITERATURE 3: JP 2024-027219 A

### SUMMARY OF THE INVENTION

### PROBLEM TO BE RESOLVED BY THE INVENTION

As described above, the conventional invention has various problems since structure for preventing the hum noise is complicated.

An objective of the present invention is to provide a means to prevent mixing of the hum noise by simple structure.

### MEANS FOR SOLVING THE PROBLEM

An electronic device of a first invention comprising: a substrate; a ground plate which has an arm which extends substantially parallel to a direction to which the substrate extends; and a screwing member which connects the substrate and the arm of the ground plate.

In the present invention, the substrate and the ground plate are connected by the screwing member. Unlike a conventional invention, since the substrate and the ground plate are not connected by a wiring, ground can be performed and mixing of a hum noise can be prevented.

The electronic device of a second invention is the electronic device of the first invention, wherein the electronic device further comprises an enclosure which stores the substrate and the ground plate is provided at the enclosure and exposes externally.

The electronic device of a third invention is the electronic device of the first invention, wherein the ground plate has a base which extends to a direction which is orthogonal to a direction to which the arm extends.

The electronic device of a fourth invention is the electronic device of the third invention, wherein the electronic device further comprises an enclosure which stores the substrate and the ground plate is provided at the enclosure and the base exposes externally.

The electronic device of a fifth invention is the electronic device of the first invention, wherein the ground plate is provided at each of both sides which are orthogonal to a longitudinal direction of an enclosure.

In the present invention, the ground plate is provided at each of both sides which are orthogonal to the longitudinal direction of the enclosure. Since a user of the electronic device grasps both sides which are orthogonal to the longitudinal direction of the enclosure and uses the electronic device, it is easy for a hand of the user to contact the ground plate.

The electronic device of a sixth invention is the electronic device of the first invention, wherein each of both sides which are orthogonal to a longitudinal direction of an enclosure is recessed toward an inside of the enclosure.

In the present invention, each of both sides which are orthogonal to the longitudinal direction of the enclosure is recessed toward the inside of the enclosure. Therefore, it is easy for a user of the electronic device to grasp the recessed part of a side wall and use the electronic device.

The electronic device of a seventh invention is the electronic device of the sixth invention, wherein the ground plate is provided at each of the recessed parts of both sides which are orthogonal to the longitudinal direction of the enclosure.

The electronic device of an eighth invention is the electronic device of the first invention, wherein a circuitry is mounted on the substrate.

The electronic device of a ninth invention is the electronic device of the first invention, wherein the electronic device further comprises a piezoelectric element.

Since a piezoelectric element is high impedance, it is easy that a hum noise mixes. In the present invention, although the electronic device comprises the piezoelectric element, mixing of the hum noise can be prevented by the ground plate.

The electronic device of a tenth invention is the electronic device of the first invention, wherein the circuitry includes an amplifier.

Since an amplifier is high impedance, it is easy that a hum noise mixes. In the present invention, although the electronic device comprises the amplifier, mixing of the hum noise can be prevented by the ground plate.

An electronic device of an eleventh invention comprising: a substrate; a piezoelectric element; and a double-core shielded wire which connects ground on the substrate and the piezoelectric element.

In the present invention, ground on the substrate and the piezoelectric element are connected by the double-core shielded wire. Thus, mixing of a hum noise can be prevented.

The electronic device of a twelfth invention is the electronic device of the first invention or eleventh invention, wherein the electronic device is a stethoscope.

### EFFECT OF THE INVENTION

According to the present invention, mixing of a hum noise can be prevented by simple constructure.

### BRIEF DESCRIPTION OF THE DRAWINGS

Fig. 1 is a perspective diagram illustrating a stethoscope according to an embodiment of the present invention.
Fig. 2 is a perspective diagram illustrating the stethoscope according to the embodiment of the present invention.
Fig. 3 is a front diagram illustrating the stethoscope according to the embodiment of the present invention.
Fig. 4 is a rear diagram illustrating the stethoscope according to the embodiment of the present invention.
Fig. 5 is a top diagram illustrating the stethoscope according to the embodiment of the present invention.
Fig. 6 is a bottom diagram illustrating the stethoscope according to the embodiment of the present invention.
Fig. 7 is a right side diagram illustrating the stethoscope according to the embodiment of the present invention.
Fig. 8 is a left side diagram illustrating the stethoscope according to the embodiment of the present invention.
Fig. 9 is a cross-sectional diagram in a direction which is orthogonal to a longitudinal direction of the stethoscope.
Fig. 10 is an enlarged diagram of a main part in Fig. 9.
Fig. 11 is a perspective diagram illustrating a ground plate.
Fig. 12 is a cross-sectional diagram in the direction which is orthogonal to the longitudinal direction of the stethoscope.
Fig. 13 is an enlarged diagram of a main part in Fig. 12.
Fig. 14 is a perspective diagram illustrating the stethoscope in a state in which an inside of the stethoscope can be seen.
Fig. 15 is a scheme diagram illustrating a state in which a piezoelectric element and a substrate are connected by a double-core shielded wire.
Fig. 16 is a diagram for describing difference of sensitivity by size difference of the piezoelectric element.
Fig. 17 is a perspective diagram illustrating a conventional stethoscope.
Fig. 18 is a perspective diagram illustrating a stethoscope according to a conventional invention.

### DESCRIPTION OF THE EMBODIMENTS

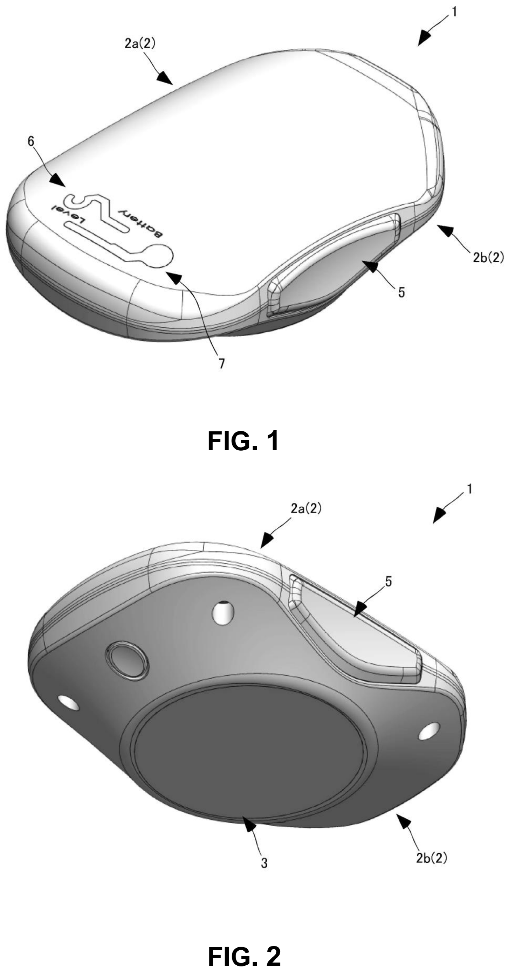

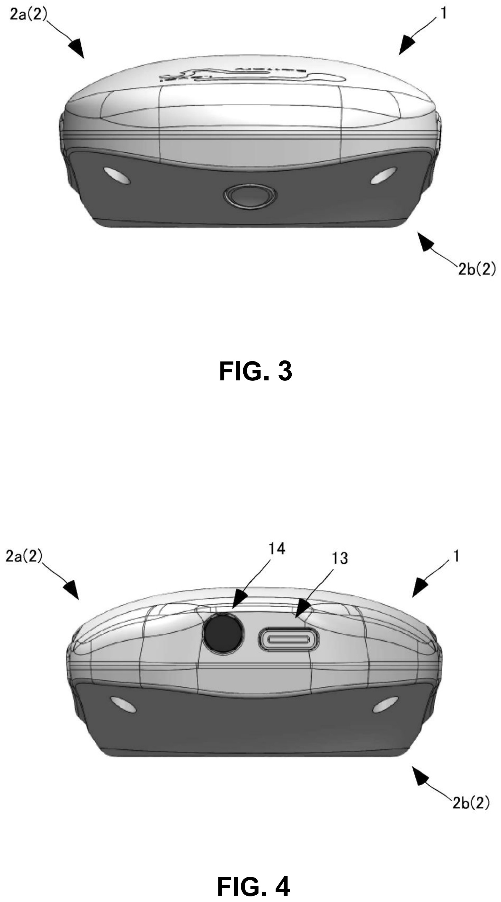

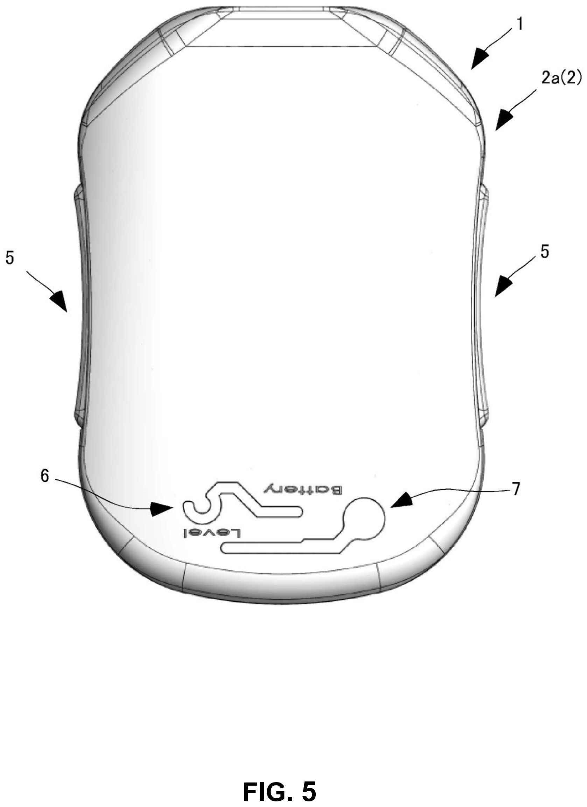

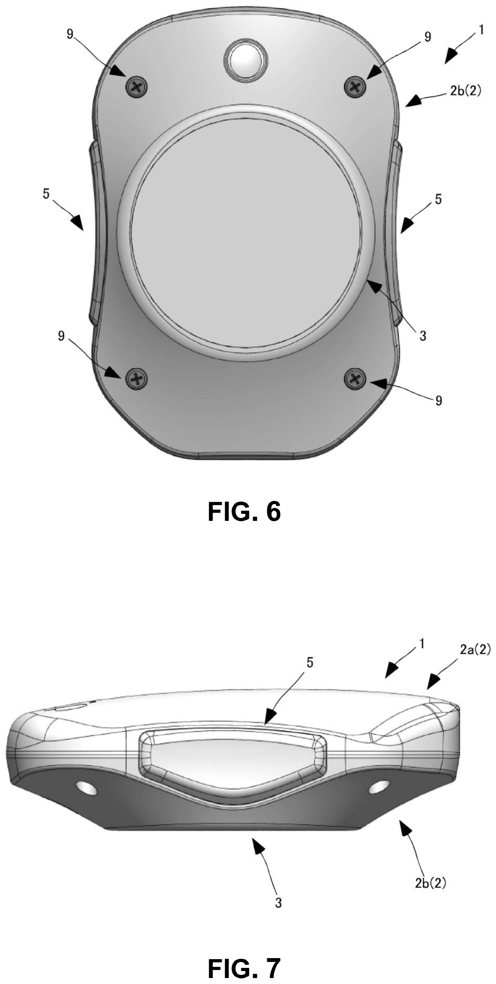

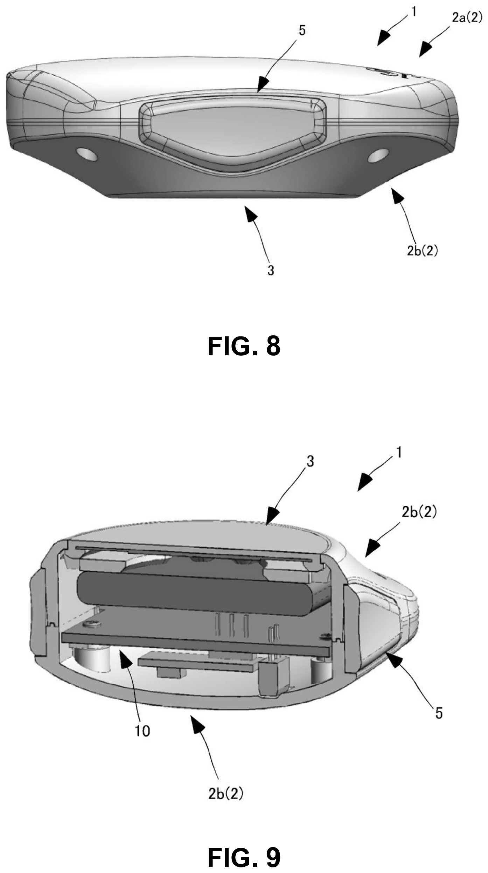

An embodiment of the present invention is described below. Each of Fig. 1 and Fig. 2 is a perspective diagram illustrating a stethoscope 1 (an electronic device) according to an embodiment of the present invention. Fig. 3 is a front diagram illustrating the stethoscope 1 according to the embodiment of the present invention. Fig. 4 is a rear diagram illustrating the stethoscope 1 according to the embodiment of the present invention. Fig. 5 is a top diagram illustrating the stethoscope 1 according to the embodiment of the present invention. Fig. 6 is a bottom diagram illustrating the stethoscope 1 according to the embodiment of the present invention. Fig. 7 is a right side diagram illustrating the stethoscope 1 according to the embodiment of the present invention. Fig. 8 is a left side diagram illustrating the stethoscope 1 according to the embodiment of the present invention.

The stethoscope 1 collects a heart sound, a lung sound and the like for auscultation. A user of the stethoscope 1 uses the stethoscope 1 by grasping the stethoscope 1 from a top surface side of the stethoscope 1 by his or her hand and contacting a bottom surface side of the stethoscope 1 to an auscultatory subject. A piezoelectric element 4 is provided inside and at the bottom surface side of the stethoscope 1.

The stethoscope 1 includes an enclosure 2, a holder 3, the piezoelectric element 4, a ground plate 5 and the like. The enclosure 2 (the stethoscope 1) is a substantially rectangular shape and four corner parts of a substantially rectangular shape are chamfered. Each of both sides which are orthogonal to a longitudinal direction of the enclosure is recessed to an inside direction. A substrate 10 and the like are stored inside the enclosure 2. The enclosure 2 consists of an upper enclosure 2a and a lower enclosure 2b. The upper enclosure 2a and the lower enclosure 2b are screwed by four screws 9. The screw 9 is inserted from the bottom surface side of the lower enclosure 2b into the upper enclosure 2a side and screws the upper enclosure 2a and the lower enclosure 2b. In the lower enclosure 2b, at the bottom surface side, an opening for which the holder 3 is provided is provided. The holder 3 exposes from the opening of the enclosure 2. The enclosure 2 has tapers. One taper is from the opening toward one direction (a front direction) in a longitudinal direction of the enclosure 2 and the other taper is from the opening toward the other direction (a rear direction) in the longitudinal direction of the enclosure 2. Namely, the enclosure 2 becomes a taper shape from the opening toward the front direction and a taper shape from the opening toward the rear direction.

At the top surface side of the stethoscope 1, a battery meter 6 which indicates the remaining capacity of a battery and a level meter 7 which indicates the level of collected sound are provided. Further, at the rear surface side of the stethoscope 1, a USB port 13 and an earphone jack 14 are provided.

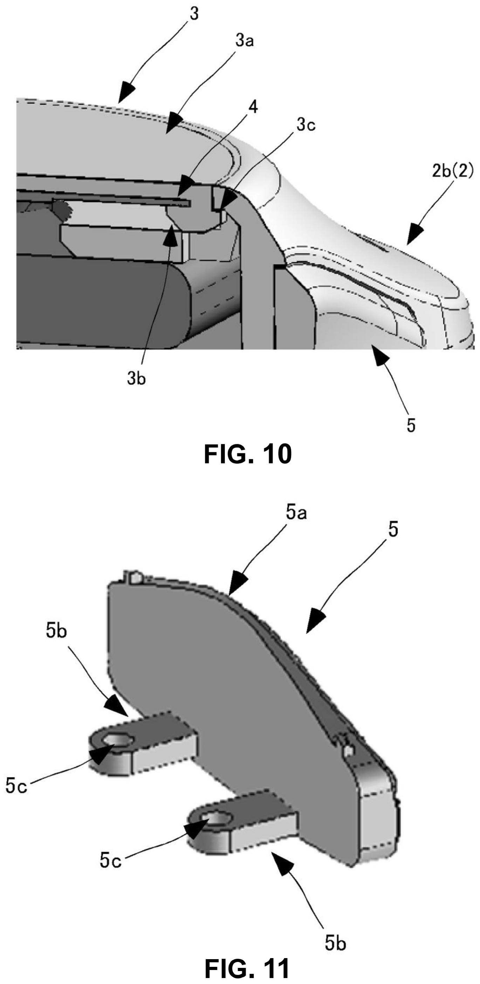

Fig. 9 is a cross-sectional diagram in a direction which is orthogonal to the longitudinal direction of the stethoscope 1. Fig. 10 is an enlarged diagram of a main part in Fig. 9. The holder 3 is for holding the piezoelectric element 4. For example, the holder 3 is made of a rubber which has elasticity. The holder 3 is circular when seen from top. The holder 3 has a base 3a, a holding part 3b and an engaging claw 3c. The base 3a is a circular shape part with a predetermined thickness. The holding part 3b is a part which holds the piezoelectric element 4. The holding part 3b bends from an outer circumference toward an inside. There is a space between the base 3a and the holding part 3b. The piezoelectric element 4 is fitted into the holding part 3b and the outer circumference of the piezoelectric element 4 is held by the holding part 3b. Namely, in a state in which the outer circumference of the piezoelectric element 4 is fitted into the space between the base 3a and the holding part 3b, the holder 3 sandwiches and holds the outer circumference of the piezoelectric element 4. The engaging claw 3c is for engaging the holder 3 with the lower enclosure 2b (the enclosure 2). The engaging claw 3c engages with an edge of the opening of the enclosure 2. The holder 3 is mounted on the lower enclosure 2b by the engaging claw 3c. For example, the six engaging claws 3c are provided at equal intervals in a circumferential direction at the holder 3 which is circular when seen from top.

The piezoelectric element 4 is flat and substantially disk-shaped. The piezoelectric element 4 has a predetermined thickness. The piezoelectric element 4 is mounted on the holder 3. In the embodiment, the holder 3 becomes a contact surface which contacts a human body (a subject). The piezoelectric element 4 converts sound (vibration) from the human body into voltage via the holder 3.

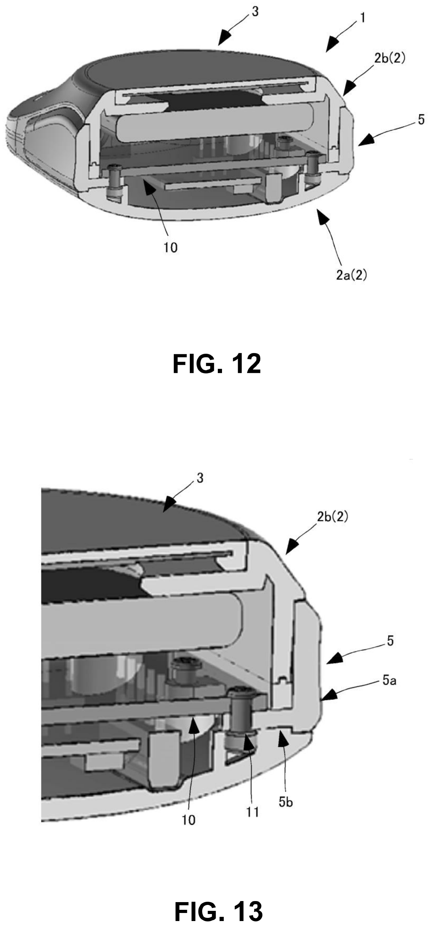

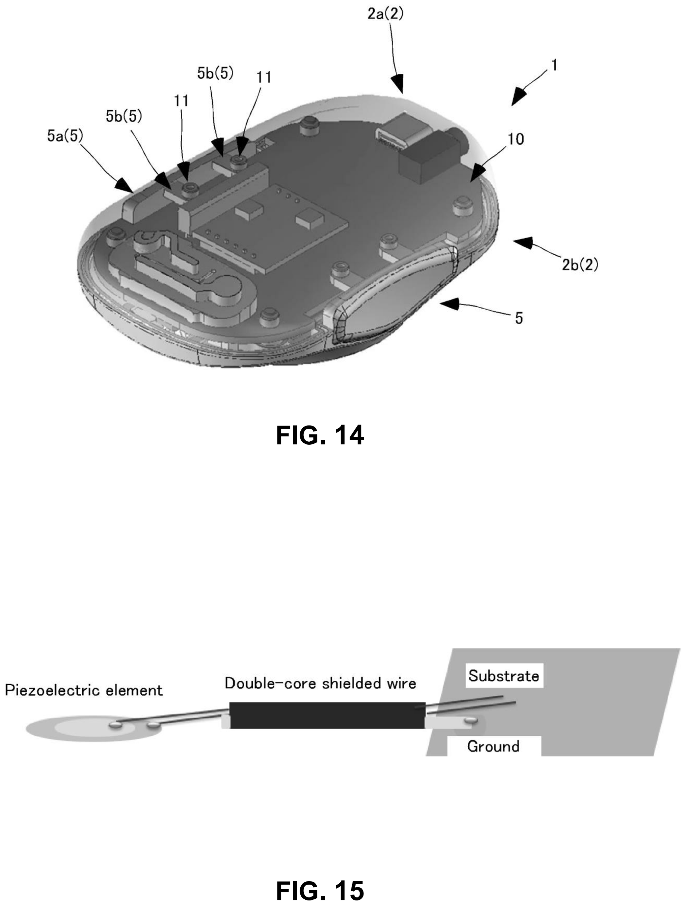

Fig. 11 is a perspective diagram illustrating the ground plate 5. Fig. 12 is a cross-sectional diagram in the direction which is orthogonal to the longitudinal direction of the stethoscope 1. Fig. 13 is an enlarged diagram of a main part in Fig. 12. Fig. 14 is a perspective diagram illustrating the stethoscope 1 in a state in which an inside of the stethoscope can be seen. Two ground plates 5 are provided at both sides of a direction which is orthogonal to the longitudinal direction of the enclosure 2 (the stethoscope 1). The ground plate 5 is provided at recessed parts of both sides which are orthogonal to the longitudinal direction of the enclosure 2. The ground plate 5 exposes from the enclosure 2 externally. The ground plate 5 is a substantially pentagonal shape when seen from top. The ground plate 5 has a base 5a and two arms 5b. The arm 5b projects from an extending direction of the base 5a to a direction perpendicular to the extending direction of the base 5a. A screw hole 5c is provided at a tip of the arm 5b. The arm 5b extends substantially parallel to a direction to which the substrate 10 extends. Namely, the arm 5b and the substrate 10 are substantially parallel each other. The ground plate 5 is fixed to the substrate 10 by a screw 11 which is inserted into the screw hole 5c. Namely, the arm 5b and the substrate 10 which are substantially parallel to each other are screwed by the screw 11 (a screwing member) which extends to a direction perpendicular to the arm 5b and the substrate 10.

Further, the piezoelectric element 4 is connected to ground on the substrate 10 by a double-core shielded wire as illustrated in Fig. 15 schematically.

The circuitry including an amplifier which is for amplifying sound which is collected by the piezoelectric element 4 is mounted on the substrate 10.

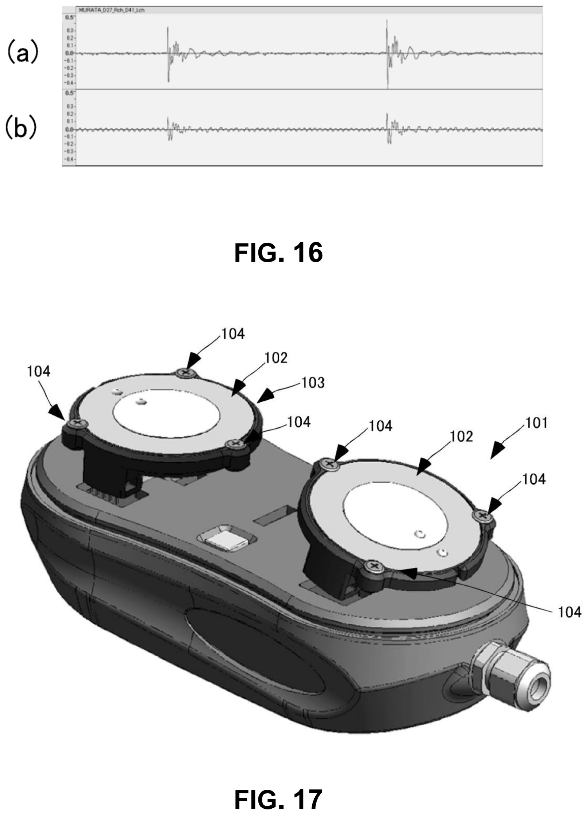

Fig. 16 is a diagram for describing difference of sensitivity by size difference of the piezoelectric element. Fig. 16(a) illustrates level of a piezoelectric element having a diameter of 41 mm. Fig. 16(b) illustrates level of a piezoelectric element having a diameter of 27 mm. Herein, a weight is mounted on a piezoelectric element which is put on ground and vibration of ground is measured by the piezoelectric element. As illustrated, level of the piezoelectric element having the diameter of 41 mm is larger than level of the piezoelectric element having the diameter of 27 mm. From this, it is understood that sensitivity of a piezoelectric element having a larger diameter is higher as compared to a smaller one.

Conventionally, as illustrated in Fig. 17, since a piezoelectric element 102 is mechanically fixed to a holder 103 by a screw 104, a space for the screw 104 is necessary at an outer circumference of the piezoelectric element 102. Further, since the screw 104 is used, there is unevenness around the piezoelectric element 102 and on a cover which covers the piezoelectric element 102. Further, in case of fixing by the screw 104, controlling of the strength of screwing the screw 104 is necessary and management of assembly is difficult.

As described above, the holder 4 has the holding part 4b which bends from the outer circumference of the base 4a to the inside of the base 4a. By the holding part 4b, the outer circumference of the piezoelectric element 3 is sandwiched and held, a bottom surface of the piezoelectric element 3 is covered by the holder 4 and falling from the stethoscope 1 is prevented. In the embodiment, since a screw is not used for mounting the piezoelectric element 3 on the holder 4 and space for screwing is not necessary, space is saved. Thus, a piezoelectric element in which sensitivity is high and a diameter is large can be used. Further, since there is not unevenness around the piezoelectric element 3 and on a surface of the holder 4 which covers the piezoelectric element 3, sound can be collected well when the surface of the holder 4 of the stethoscope 1 is contacted to the human body which is an auscultatory subject. Further, since a screw is not used, controlling of the strength of screwing the screw is not necessary and assemblability is improved.

The holder 4 has an engaging claw 4c which projects from the outer circumference of the base 4a. The engaging claw 4c is engaged with the edge of the opening of the enclosure 2 (the lower enclosure 2b) and the piezoelectric element 3 which is held by the holder 4 is mounted on the enclosure 2 (the lower enclosure 2b). Further, for collecting sound, it is important that the piezoelectric element 3 (the holder 4) contacts the human body which is the auscultatory subject. By saving space, it is easy that the holder 4 (the piezoelectric element 3) follows unevenness of the human body which is the auscultatory subject and fits to the human body. In addition, since a screw is not used for mounting the holder 4 on the enclosure 2, the number of parts is increased and assemblability of the stethoscope 1 is improved.

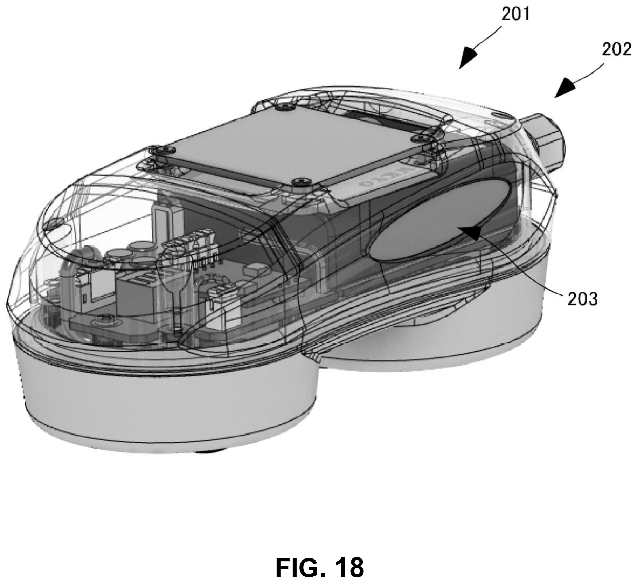

Conventionally, as illustrated in Fig. 18, in a stethoscope 201, a ground plate 203 is provided so that the ground plate 203 exposes from the enclosure 202. Wiring against the ground plate is a structure that a wiring is soldered to an inside of a sheet metal member and the wiring is connected to an internal substrate. According to this structure, the wiring may be disconnected or bitten and assemblability may be spoiled. Further, since the sheet metal is used, there is limitation for design.

In the embodiment, since the ground plate 5 contacts the substrate 10 by the arm 5b directly and is securely fixed to the substrate 10 by the screw 11, thereby ensuring reliable grounding. Further, since the ground plate 5 is provided at each of both sides which are orthogonal to the longitudinal direction of the enclosure 2 and the part which is recessed to the inside, the user's hand contacts the ground plate 5 naturally when using the stethoscope 1 and ground can be performed certainly. Further, it is easy for the user to grasp the recessed part.

Further, conventionally, since wiring of a piezoelectric element becomes a loop shape like an antenna and is easy to pick noise up, it is necessary to care about a wiring route. Further, in wiring, since impedance is high, there is a case where noise superimposes by electrical combination when the wiring is naked.

In the embodiment, the piezoelectric element 4 is connected to ground on the substrate 10 by the double-core shielded wire. Thus, mixing of external noise is prevented.

As described above, in the embodiment, the piezoelectric element 4 is sandwiched and held by the holder 3. Thus, without using a screw or the like, the piezoelectric element 4 can be mounted on the stethoscope 1 by simple structure.

Further, the holder 3 which holds the piezoelectric element 4 has the engaging claw 3c which is engaged with the enclosure 2. Thus, since the holder 3 which holds the piezoelectric element 4 is mounted on the enclosure 2 of the stethoscope 1 by the engaging claw 3c, the piezoelectric element 4 can be mounted on the stethoscope 1 by simple structure without using a screw or the like.

Further, in the embodiment, the holder 3 which holds the piezoelectric element 4 has the holding part 3b which bends from the outer circumference to the inside of the base 3a. Thus, the piezoelectric element 4 is fitted into the space between the holding part 3b and the base 3a and held by the holder 3. Further, the holder 3 has the engaging claw 3c which projects from the outer circumference of the base 3a to the outside of the base 3a. Therefore, the holder 3 which holds the piezoelectric element 4 is mounted on the enclosure 2 by the engaging claw 3c. Thus, in the embodiment, the piezoelectric element 4 can be mounted on the stethoscope 1 by simple structure without using a screw or the like.

In a state of collecting sound by the piezoelectric element 4, the holder 3 which exposes from the opening of the enclosure 2 becomes a contact surface for contact a subject from which sound is collected. In the embodiment, the enclosure 2 has tapers. One taper is toward one direction (the front direction) of the longitudinal direction of the enclosure 2 and the other taper is toward the other direction (the rear direction) of the longitudinal direction of the enclosure 2. Therefore, even if the subject from which sound is collected has unevenness when using the stethoscope 1, it is easy to contact the contact surface for contact the subject.

Further, in the embodiment, the substrate 10 and the ground plate 5 are screwed by the screw 11. Unlike the conventional invention, since a substrate and a ground plate are not connected by a wiring, ground can be performed by simple structure and mixing of the hum noise can be prevented.

Further, in the embodiment, each of both sides which are orthogonal to the longitudinal direction of the enclosure 2 is recessed to the inside. Therefore, it is easy for the user of the stethoscope 1 to use the stethoscope 1 by grasping the recessed part of a side wall.

Since a piezoelectric element is high impedance, it is easy that the hum noise mixes. In the embodiment, although the stethoscope 1 includes the piezoelectric element 4, mixing of the hum noise can be prevented by the ground plate 5.

Since an amplifier is high impedance, it is easy that the hum noise mixes. In the embodiment, although the stethoscope 1 includes the amplifier, mixing of the hum noise can be prevented by the ground plate 5.

Further, ground on the substrate and the piezoelectric element are connected by the double-core shielded wire. Thus, mixing of the hum noise can be prevented.

The embodiment of the present invention is described above, but the mode to which the present disclosure is applicable is not limited to the above embodiment and can be suitably varied without departing from the scope of the present disclosure.

### INDUSTRIAL APPLICABILITY

The present invention can be suitably employed in an electronic device.

### DESCRIPTION OF REFERENCE SIGNS

- 1: stethoscope (electronic device)
- 2: enclosure
- 2a: upper enclosure
- 2b: lower enclosure
- 3: holder
- 3a: base
- 3b: holding part
- 3c: engaging claw
- 4: piezoelectric element
- 5: ground plate
- 5a: base
- 5b: arm
- 5c: screw hole
- 10: substrate
- 11: screw (screwing member)

## Claims

1. An electronic device (1) comprising:
a substrate (10);
a ground plate (5) which has an arm (5b) which extends substantially parallel to a direction to which the substrate (10) extends; and
a screwing member (11) which connects the substrate (10) and the arm (5b) of the ground plate (5).

2. The electronic device (1) according to claim 1, further comprising:
an enclosure (2) which stores the substrate (10),
wherein the ground plate (5) is provided at the enclosure (2) and exposes externally.

3. The electronic device (1) according to claim 1,
wherein the ground plate (5) has a base which extends to a direction which is orthogonal to a direction to which the arm (5b) extends.

4. The electronic device (1) according to claim 3, further comprising: an enclosure (2) which stores the substrate (10),
wherein the ground plate (5) is provided at the enclosure (2) and the base of the ground plate (5) exposes externally.

5. The electronic device (1) according to claim 1,
wherein the ground plate (5) is provided at each of both sides which are orthogonal to a longitudinal direction of an enclosure (2).

6. The electronic device (1) according to claim 1,
wherein each of both sides which are orthogonal to a longitudinal direction of an enclosure (2) is recessed toward an inside.

7. The electronic device (1) according to claim 6,
wherein the ground plate (5) is provided at each of the recessed parts of both sides which are orthogonal to the longitudinal direction of the enclosure.

8. The electronic device (1) according to claim 1,
wherein a circuitry is mounted on the substrate (10).

9. The electronic device (1) according to claim 1, further comprising: a piezoelectric element (4).

10. The electronic device (1) according to claim 1,
wherein the circuitry includes an amplifier.

11. An electronic device (1) comprising:
a substrate (10);
a piezoelectric element (4); and
a double-core shielded wire which connects ground on the substrate (10) and the piezoelectric element (4).

12. The electronic device (1) according to claim 1 or 11,
wherein the electronic device (1) is a stethoscope.
